# FASCICULE DE BREVET EUROPEEN

(11) **EP 0 803 253 B1**
(45) Date de publication et mention de la délivrance du brevet: **19.09.2001**
(21) Numéro de dépôt: 97400911.0
(22) Date de dépôt: 23.04.1997
(51) Int. Cl.: A61K 31/554, A61K 9/20

(54) **Comprimés matriciels à utilisation orale et à libération prolongée contenant le sel de sodium de tianeptine**
Oral anzuwendende Matrixtabletten mit verzögerter Wirkstoffabgabe enthaltend Tianeptin Natriumsalz
Matrix-type oral sustained-release tablets containing tianeptine sodium salt

(30) Priorité: 24.04.1996 FR 9605174
(43) Date de publication de la demande: 29.10.1997
(73) Titulaire: ADIR ET COMPAGNIE, 92415 Courbevoie Cédex (FR)
(72) Inventeur: Huet De Barochez, Bruno, 45140 Ingre (FR); Wuthrich, Patrick, 45000 Orleans (FR)

(56) Documents cités:
- EP-A- 0 671 173
- FR-A- 2 635 461

## Description

La présente invention a pour objet un comprimé matriciel permettant la libération prolongée du sel de sodium de tianeptine, assurant des taux sanguins réguliers et constants après absorption de la forme galénique par voie orale.

Le sel de sodium de tianeptine, composé de formule (I): 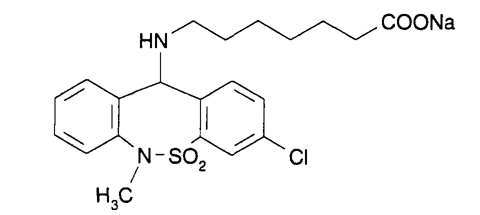 est un antidépresseur tricyclique. La molécule utilisée pour l'administration à l'homme est le sel de sodium.

Le sel de sodium de tianeptine était jusqu'à présent administré par voie orale sous forme de comprimés à libération immédiate dosés à 12,5 mg. La prescription moyenne habituelle est de trois prises par jour.

Or, une forme à libération immédiate peut conduire, chez certains sujets, à l'obtention de pics sanguins importants. Une forme à libération prolongée permet d'éviter ces pics sanguins et d'obtenir une concentration sanguine régulière chez l'homme. Ceci permet de réduire les effets indésirables pouvant éventuellement survenir par "effet de pic" accompagnés de troubles de type hydroélectrolytiques et métabolites reliés aux variations des taux plasmatiques du principe actif.

Une forme à libération prolongée du sel de sodium de tianeptine permet donc d'assurer un meilleur index thérapeutique dans le traitement de l'anxiété et de la dépression.

Pour ce faire, il est nécessaire d'assurer une libération prolongée dans le temps, de façon parfaitement contrôlée. La vitesse de libération doit être reproductible et corrélée avec les concentrations sanguines observées après administration.

Parmi les mécanismes pouvant être invoqués pour le contrôle de la diffusion d'un principe actif soluble, on peut en retenir un principal, qui est la diffusion du principe actif au travers d'un gel formé après gonflement d'un polymère hydrophile mis au contact du liquide de dissolution (in vitro), ou du liquide gastro-intestinal (in vivo).

De nombreux polymères ont été décrits comme pouvant permettre la formation de ce gel. Les principaux sont les dérivés de la cellulose, en particulier les éthers de cellulose tels l'hydroxy-propylcellulose, l'hydroxyméthylcellulose, la méthylcellulose, la méthylhydroxypropylcellulose, et parmi les différentes qualités commerciales de ces éthers celles présentant des viscosités assez élevées. II faut noter que les systèmes décrits ne permettent pas théoriquement d'atteindre un ordre zéro dans l'équation de la cinétique de libération.

Les procédés de fabrication couramment utilisés pour la fabrication de tels comprimés matriciels sont soit la compression directe, après mélange des différents excipients et du ou des principes actifs, soit la granulation humide.

Le comprimé matriciel décrit dans la présente invention associe de façon originale un polymère dérivé de la cellulose et un sel de calcium, cette association permettant d'obtenir une libération du principe actif parfaitement contrôlée. En outre, cette association s'adapte parfaitement aux caractéristiques physico-chimiques du sel de sodium de la tianeptine.

Cette libération contrôlée est quasiment linéaire pendant plus de huit heures et est telle que 50 pour cent de la quantité totale du sel de sodium de tianeptine est libérée entre 5 et 14 heures. D'autre part le comprimé matriciel selon l'invention permet d'obtenir une libération prolongée de tianeptine conduisant à des taux sanguins chez l'homme compris entre 50 et 300 ng/ml, 24 heures au plus après administration du comprimé. La posologie unitaire peut ainsi varier selon l'âge et le poids du patient, la nature et la sévérité de l'affection. D'une manière générale, elle s'échelonne entre 12,5 et 50 mg pour un traitement journalier.

Le polymère utilisé est une méthylhydroxypropylcellulose de haute viscosité, le sel minéral est l'hydrogénophosphate de calcium dihydraté. L'association de ces deux composés permet d'obtenir un parfait contrôle de la cinétique de libération. Le pourcentage de polymère dérivé de la cellulose est compris entre 30 et 50 % de la masse totale du comprimé, le pourcentage de sel minéral est compris entre 10 et 60 % de la masse totale du comprimé. Différents excipients peuvent également être ajoutés pour la finalisation du comprimé.

La présente invention concerne également la préparation de ce comprimé matriciel. Une granulation humide est effectuée avec le principe actif, le sel minéral et du lactose, afin de créer autour du principe actif un environnement hydrophile favorable à sa bonne dissolution, et également de manière à obtenir un dosage unitaire le plus régulier possible. Cependant, l'adjonction d'un liant n'est pas nécessaire dans cette formulation pour obtenir un comprimé régulier. Après cette étape de granulation, un mélange de compression directe est réalisé, puis comprimé.

Les exemples suivants illustrent l'invention. La préparation des comprimés à libération prolongée est réalisée selon le procédé de fabrication suivant :

### Stade A :

Mélange du sel de sodium de tianeptine, de l'hydrogénophosphate de calcium dihydraté et du lactose, puis mouillage de ce mélange au moyen d'eau purifiée ou d'une solution hydro-alcoolique. La masse humide préparée est ensuite granulée, séchée puis calibrée, de manière à obtenir un granulé dont les caractéristiques physiques permettent un bon remplissage des matrices d'une machine à comprimer rapide.

### Stade B :

Mélange du granulé obtenu au stade A avec la méthylhydroxypropylcellulose.

### Stade C :

Lubrification du mélange obtenu au stade B avec de la silice colloïdale et du stéarate de magnésium.

### Stade D :

Compression du mélange lubrifié obtenu au stade C sur machine à comprimer rotative, de manière à obtenir des comprimés ayant une dureté mesurée par écrasement diamétral d'environ 6 à 12 daN.

### EXEMPLE 1 :

L'exemple 1 montre l'influence des constituants sur la cinétique de libération *in vitro*. Un plan d'expérience est mis en oeuvre dans lequel la quantité de méthylhydroxypropylcellulose varie de 80 à 120 mg par comprimé, la quantité d'hydrogénophosphate de calcium dihydraté de 0 à 146,4 mg par comprimé et la quantité de lactose de 146,4 à 0 mg par comprimé. La quantité totale de diluant (lactose + hydrogénophosphate de calcium dihydraté) est de 146,4 mg par comprimé.

La fabrication est réalisée suivant le procédé opératoire décrit dans les stades A à D.

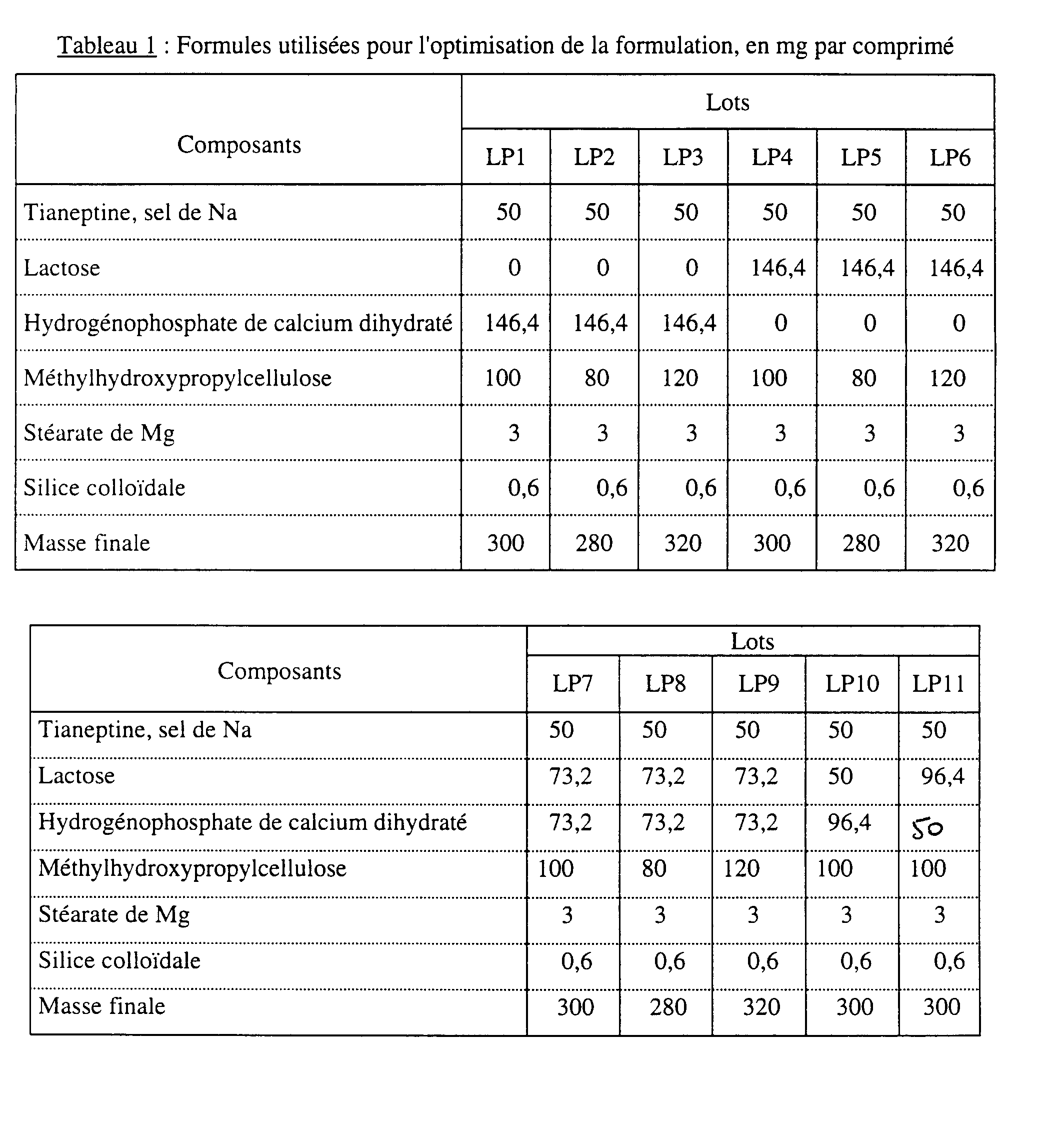

Le tableau 2 montre les pourcentages de principe actif dissous à 8 h en fonction des formulations utilisées.

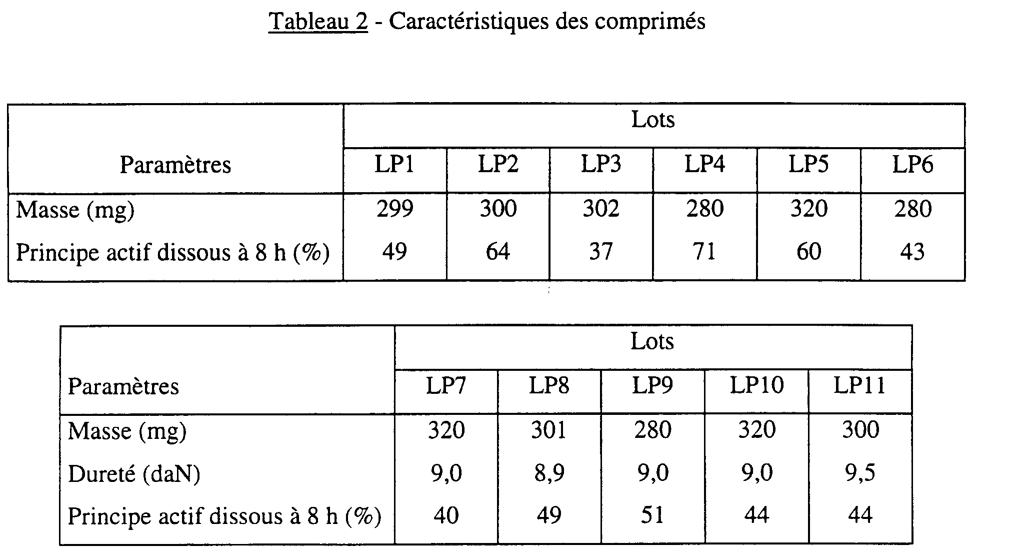

Sur les figures 1 et 2 sont représentées les courbes de cinétique de dissolution pour les différentes formulations utilisées.

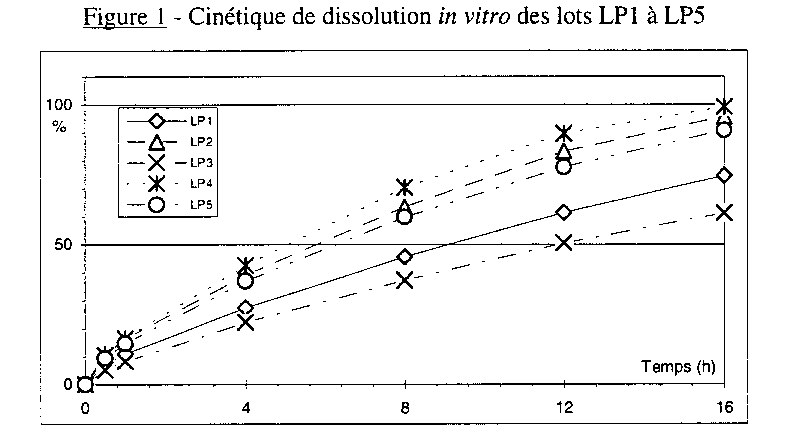

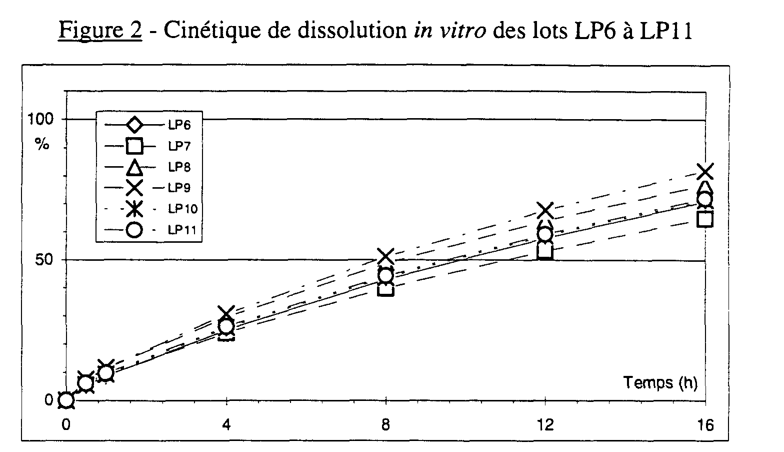

Ces courbes montrent nettement que les pourcentages de méthylhydroxypropylcellulose et d'hydrogénophosphate de calcium dihydraté jouent un rôle important dans le contrôle de la cinétique de dissolution *in vitro.* L'augmentation de la méthylhydroxypropylcellulose de 80 à 120 mg diminue le pourcentage de principe actif dissous à 8 h d'environ 7. La substitution totale du lactose par de l'hydrogénophosphate de calcium dihydraté diminue ce même pourcentage de près de 25.

### EXEMPLE 2 :

Un comprimé à libération prolongée (LP 12) est préparé en utilisant la formule donnée dans le tableau 3, suivant le procédé opératoire décrits dans les stades A à D.

**Tableau 3 :**

| Formule unitaire du comprimé LP 12 | |
|---|---|
| Constituants | Quantités (mg) |
| Tianeptine sel de sodium | 50 |
| Hydrogénophosphate de calcium dihydraté | 50 |
| Lactose monohydraté | 96,4 |
| Méthylhydroxypropylcellulose | 100 |
| Silice colloïdale anhydre | 0,6 |
| Stéarate de magnésium | 3 |
| Enrobage blanc | 15 |

Le profil de dissolution in vitro de cette forme (LP 12) est présenté dans la figure ci dessous.

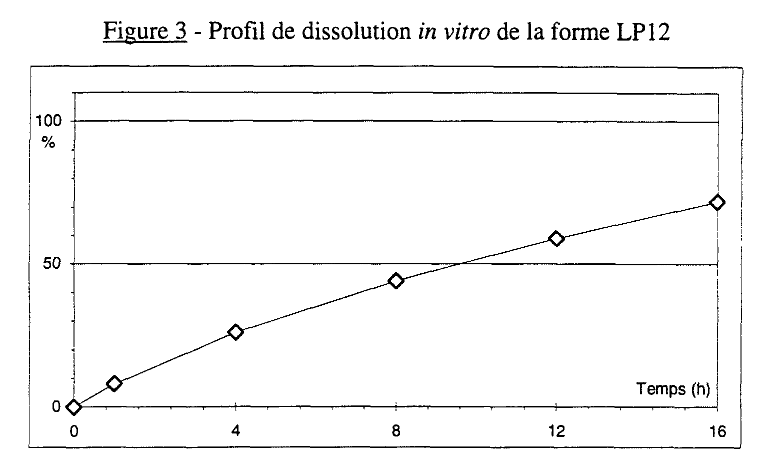

La cinétique plasmatique de tianeptine sel de sodium est mesurée chez quatre sujets après administration unique d'un comprimé LP12. La concentration plasmatique moyenne est donnée dans la figure suivante.

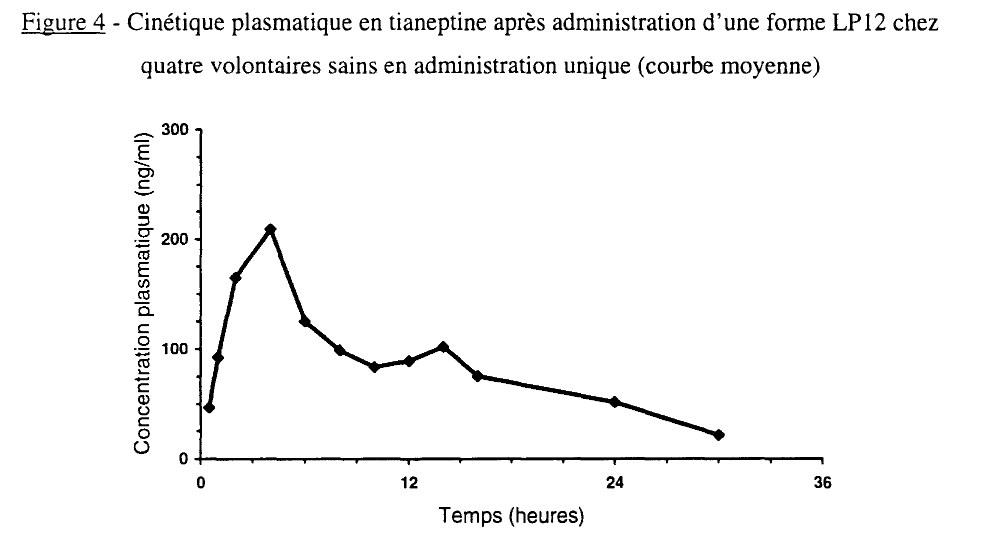

Cette courbe montre un profil de dissolution de type matriciel (libération continue du principe actif), avec une cinétique plasmatique biphasique (deux pics plasmatiques).

### EXEMPLE 3 :

Deux comprimés à libération prolongés LP13 et LP14 sont préparés en utilisant les formules donnée dans le tableau 2, suivant le procédé opératoire décrit dans les stades A à D. Deux qualités de méthylhydroxypropylcellulose sont utilisées de manière à préparer un lot à dissolution lente et un lot à dissolution plus rapide.

**Tableau 4 :**

| Formule unitaire des comprimés LP13 et LP14 | | |
|---|---|---|
| Constituants | Comprimé LP13 (mg) | Comprimé LP14 (mg) |
| Tianeptine sel de sodium | 50 | 50 |
| Hydrogénophosphate de calcium dihydraté | 74 | 74 |
| Lactose monohydraté | 74 | 74 |
| Méthylhydroxypropylcellulose 2208 40 Ps | 100 | 82 |
| Méthylhydroxypropylcellulose 2208 1 Ps | 0 | 18 |
| Silice colloïdale anhydre | 0,5 | 0,5 |
| Stéarate de magnésium | 1,5 | 1,5 |
| Enrobage blanc | 5 | 5 |

Le profil de dissolution in vitro de cette forme est présenté dans la figure ci après.

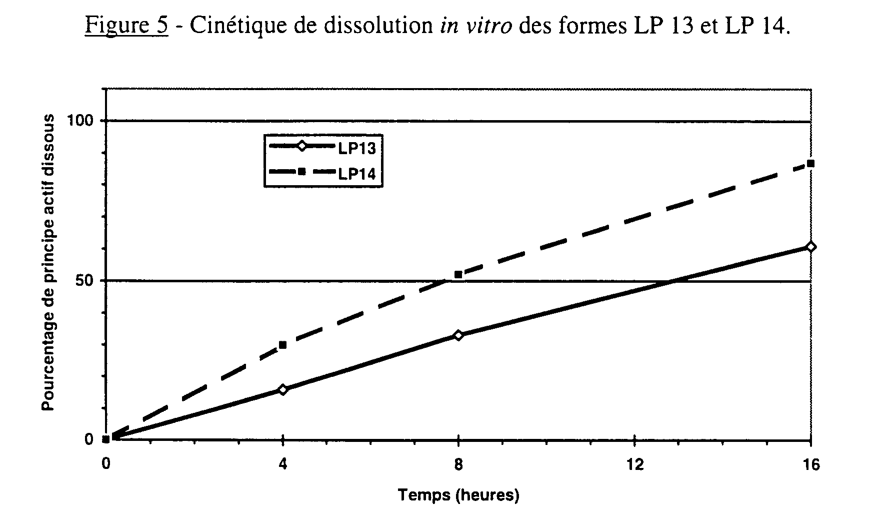

La cinétique plasmatique de tianeptine sel de sodium est mesurée chez huit sujets après administration unique d'un comprimé LP13 ou LP14 en cross-over. La concentration plasmatique moyenne est donnée dans la figure suivante.

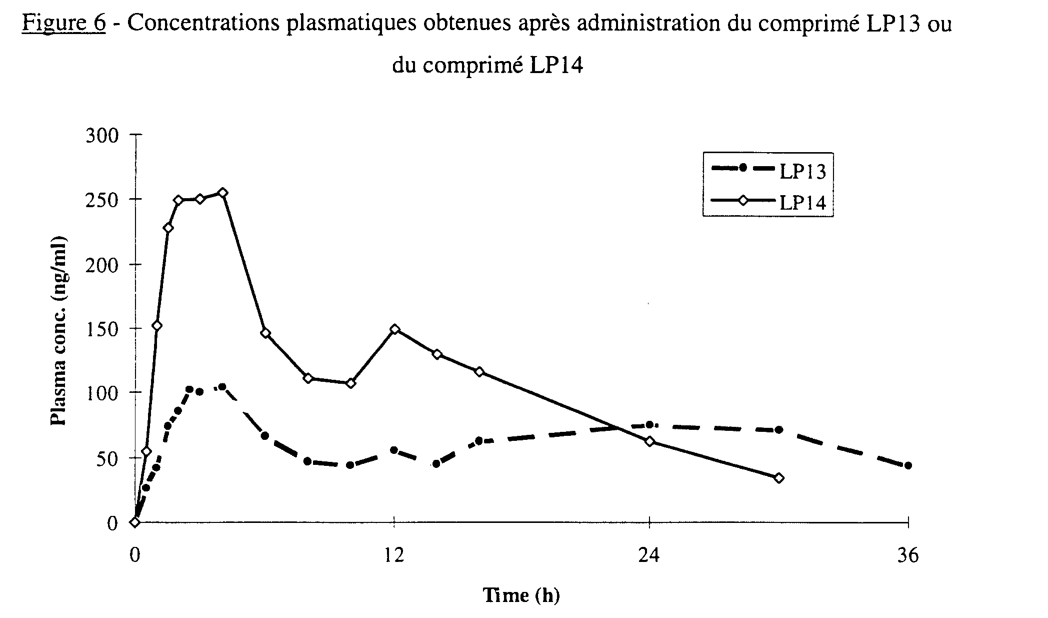

La comparaison entre les formules LP13 et LP14 ne différant que par la qualité de méthylhydroxypropylcellulose utilisée montre que l'on peut contrôler également la cinétique de dissolution in vitro de tianeptine sel de sodium par ce moyen. La relation avec la cinétique sanguine mesurée in vivo est très bonne. Les deux cinétiques sanguines sont significativement différentes. On retrouve les deux pics de libération du principe actif.

## Revendications

1. Comprimé matriciel pour la libération prolongée du sel de sodium de tianeptine **caractérisé en ce que** cette libération prolongée est contrôlée par l'utilisation d'un polymère dérivé de la cellulose et d'un sel de calcium.

2. Comprimé matriciel de tianeptine selon la revendication 1 **caractérisé en ce que** le polymère dérivé de la cellulose est une méthylhydroxypropylcellulose.

3. Comprimé matriciel de tianeptine selon la revendication 1 **caractérisé en ce que** le sel de calcium est de l'hydrogénophosphate de calcium dihydraté.

4. Comprimé matriciel de tianeptine selon la revendication 1 **caractérisé en ce que** les pourcentages de dérivé de cellulose et de sel de calcium sont respectivement compris entre 30 et 50 % et entre 10 et 60 % de la masse totale du comprimé.

5. Comprimé matriciel de tianeptine selon la revendication 1 **caractérisé en ce que** les pourcentages de dérivé de cellulose et de sel de calcium permettent une libération prolongée de tianeptine conduisant à des taux sanguins chez l'homme compris entre 50 et 300 ng/ml, 24 heures au plus après administration du comprimé par voie orale.

6. Procédé de préparation d'un comprimé matriciel de tianeptine selon la revendication 1 **caractérisé en ce que** l'on utilise à la fois une technique de granulation humide et de compression directe comprenant les étapes suivantes :
- stade A : mélange du sel de sodium de tianeptine, de l'hydrogénophosphate de calcium dihydraté et du lactose, puis mouillage de ce mélange au moyen d'eau purifiée ou d'une solution hydro-alcoolique, la masse humide préparée est ensuite granulée, séchée puis calibrée, de manière à obtenir un granulé dont les caractéristiques physiques permettent un bon remplissage des matrices d'une machine à comprimer rapide,
- stade B : mélange du granulé obtenu au stade A avec la méthylhydroxypropylcellulose,
- stade C : lubrification du mélange obtenu au stade B avec de la silice colloïdale et du stéarate de magnésium,
- stade D : compression du mélange lubrifié obtenu au stade C sur machine à comprimer rotative, de manière à obtenir des comprimés ayant une dureté mesurée par écrasement diamétral d'environ 6 à 12 daN.

7. Comprimé matriciel de tianeptine selon la revendication 1 utile dans le traitement de la dépression.

## Claims

1. Matrix tablet for the prolonged release of the sodium salt of tianeptine, **characterised in that** the prolonged release is controlled by the use of a cellulose derivative polymer and a calcium salt.

2. Tianeptine matrix tablet according to claim 1, **characterised in that** the cellulose derivative polymer is a hydroxypropyl methyl cellulose.

3. Tianeptine matrix tablet according to claim 1, **characterised in that** the calcium salt is calcium hydrogen phosphate dihydrate.

4. Tianeptine matrix tablet according to claim 1, **characterised in that** the percentages of cellulose derivative and calcium salt are from 30 to 50 % and from 10 to 60 %, respectively, of the total mass of the tablet.

5. Tianeptine matrix tablet according to claim 1, **characterised in that** the percentages of cellulose derivative and calcium salt allow prolonged release of tianeptine resulting in blood levels in humans of from 50 to 300 ng/ml up to 24 hours after administration of the tablet by the oral route.

6. Process for the preparation of a tianeptine matrix tablet according to claim 1, **characterised in that** there is used a technique of both wet granulation and direct compression comprising the following steps :
- step A : mixture of the sodium salt of tianeptine, calcium hydrogen phosphate dihydrate and lactose and then wetting of that mixture with purified water or an aqueous-alcoholic solution; the wet mass prepared is then granulated, dried and subsequently classified so as to obtain a granulate the physical characteristics of which enable the dies of a high-speed tabletting machine to be filled well;
- step B : mixture of the granulate obtained in step A with hydroxypropyl methyl cellulose;
- step C : lubrication of the mixture obtained in step B, using colloidal silica and magnesium stearate;
- step D : compression of the lubricated mixture obtained in step C on a rotary tablet machine so as to obtain tablets having a hardness of about from 6 to 12 daN, measured by breaking across a diameter.

7. Tianeptine matrix tablet according to claim 1 for use in the treatment of depression.

## Patentansprüche

1. Matrixtablette mit verzögerter Freisetzung des Natriumsalzes von Tianeptin, **dadurch gekennzeichnet, daß** diese verzögerte Freisetzung durch die Verwendung eines von Cellulose abgeleiteten Polymers und eines Calciumsalzes gesteuert wird.

2. Tiancptin-Matrixtablette nach Anspruch 1, **dadurch gekennzeichnet, daß** das von Cellulose abgeleitete Polymer eine Methylhydroxypropylcellulose ist.

3. Tianeptin-Matrixtablette nach Anspruch 1, **dadurch gekennzeichnet, daß** das Calciumsalz Calciumhydrogenphosphat-Dihydrat ist.

4. Tianeptin-Matrixtablette nach Anspruch 1, **dadurch gekennzeichnet, daß** die Prozentsätze von Cellulosederivat und Calciumsalz zwischen 30 und 50 % bzw, zwischen 10 und 60 % der Gesamtmasse der Tablette liegen.

5. Tianeptin-Matrixtablette nach Anspruch 1, **dadurch gekennzeichnet, daß** die Prozentsätze von Cellulosederivat und Calciumsalz eine verlängerte Freisetzung von Tianeptin ermöglichen, die beim Menschen zu Blutspiegeln zwischen 50 und 300 ng/ml 24 Stunden nach der Verabreichung der Tablette auf oralem Wege ergeben.

6. Verfahren zur Herstellung einer Tlaneptin-Matrixtablette nach Anspruch 1, **dadurch gekennzeichnet, daß** man gleichzeitig eine Technik der Feuchtgranulation und des direkten Verpressens anwendet, welche die folgenden Stufen umfaßt:
- Stufe A : Vermischen des Natriumsalzes von Tianeptin. Calciumhydrogenphosphat-Dihydrat und Lactose. Anfeuchten der Mischung mit gereinigtem Wasser oder einer wäßrig-alkoholischen Lösung, anschließendes Granulieren der in dieser Weise hergestellten feuchten Masse, Trocknen und Kalibrieren, derart, daß man ein Granulat erhält, dessen physikalische Eigenschaften eine gute Füllung der Matrix bei Anwendung einer schnellen Preßvorrichtung ermöglichen,
- Stufe B : Vermischen des in der Stufe A erhaltenen Granulats mit Methylhydroxypropylcellulose,
- Stufe C: Gleitmittelbehandlung der in der Stufe B erhaltenen Mischung mit kolloidalem Siliciumdioxid und Magnesiumstearat,
- Stufe D : Verpressen der in der Stufe C erhaltenen gleitmittelhaltigen Mischung mit einer Rotationspreßvorrichtung, derart, daß man Tabletten erhält mit einer durch diametrales Aufbrechen gemessenen Härte von etwa 6 bis 12 daN.

7. Tianepttn-Matrixtablette nach Anspruch 1 zur Behandlung von Depressionen.
